# FASCICULE DE BREVET EUROPEEN

(11) **EP 0 323 656 B1**
(45) Date de publication et mention de la délivrance du brevet: **07.04.1993**
(21) Numéro de dépôt: 88202679.2
(22) Date de dépôt: 25.11.1988
(51) Int. Cl.: C08G 61/12, H01B 1/12, C07D 333/16

(54) **Polymères conducteurs dérivés d'hétérocycles aromatiques substitués par un groupement de type éther, procédé pour leur obtention, dispositifs contenant ces polymères et monomères permettant d'obtenir de tels polymères**
Leitfähige Polymere von mit Äthergruppierung substituierten aromatischen heterocyclischen Verbindungen, Verfahren zu deren Herstellung, Vorrichtung enthaltend diese Polymere, und Monomere die ermöglichen solche Polymere zu gewinnen
Conductive polymers from heterocyclic aromatic compounds substitued with an ether group, process for their obtention, device containing these polymers, and monomers allowing to obtain such polymers

(30) Priorité: 07.12.1987 FR 8717117; 01.09.1988 FR 8811572
(43) Date de publication de la demande: 12.07.1989
(73) Titulaire: SOLVAY, B-1050 Bruxelles (BE)
(72) Inventeur: Roncali, Jean, F-93260 Les Lilas (FR); Garreau, Robert, F-95200 Sarcelles (FR); Delabouglise, Didier, F-76000 Rouen (FR); Lemaire, Marc, F-92000 Nanterre (FR); Garnier, Francis, F-94500 Champigny (FR); Hannecart, Etienne, B-1980 Tervueren (BE)
(74) Mandataire: Nichels, William

(56) Documents cités:
- EP-A- 0 096 612
- EP-A- 0 240 063
- EP-A- 0 253 594
- DE-A- 3 701 495
- US-A- 3 574 072
- US-A- 4 543 402
- JOURNAL OF THE ELECTROCHEMICAL SOCIETY, vol. 131, no. 6, Juin 1986, pages 1452-1456, Manchster, New Hampshire, US; R.J. WALTMAN et al.: "Electroactive properties of polyaromatic molecules"

## Description

La présente invention concerne des polymères conducteurs d'électricité dérivés d'hétérocycles aromatiques substitués par un groupement de type éther.

Elle concerne plus particulièrement des polymères dérivés d'hétérocycles aromatiques à cinq chaînons contenant un hétéroatome et substitués en position 3 par un substituant de type éther lié à l'hétérocycle par un radical alkylène.

L'invention concerne également un procédé pour la fabrication de ces polymères.

Elle concerne aussi des dispositifs électroconducteurs contenant ces polymères.

Enfin l'invention concerne également des monomères utilisables pour l'obtention de polymères conducteurs selon l'invention.

On a déjà décrit des électrodes obtenues par polymérisation électrochimique, sur un support conducteur, de monomères hétérocycliques possédant au moins un hétérocycle aromatique à 5 chaînons, contenant un seul hétéroatome et substitué par au moins un groupement notamment de type alkyle, alkoxyle, aryle, aryle substitué, halogène, cyano, amino ou dialkylamino. Ce monomère peut être un dérivé substitué en position 3, en position 4 ou en position 3 et 4 du pyrrole, du thiophène ou du furane, ou un indole substitué sur le noyau phényle par 1 à 4 groupements (demande de brevet FR-A-2527843).

On a aussi décrit les caractéristiques électrochromiques de polymères dérivés de ces hétérocycles à cinq chaînons, en particulier de ceux dérivés du pyrrole, du thiophène, du 3-méthylthiophène, du 3,4-diméthylthiophène et du 2,2′-dithiophène (F. Garnier et al, Journal of Electroanalytical Chemistry, 148, 1983, pages 299 à 303).

Certaines applications électriques, telles que la réalisation de dispositifs (écrans d'affichage, commutateurs, éléments de mémoire ...) basés sur l'électrochromisme (impliquant une modification des propriétés d'absorption ou de transmission de la lumière du matériau mis en oeuvre, induite par une variation de la tension externe appliquée), d'électrodes de batteries rechargeables, de cellules photovoltaîques, de cellules électrochimiques, etc. exigent des polymères conducteurs aux propriétés particulières.

Ces propriétés particulières sont notamment la réversibilité électrochimique la plus complète et la stabilité la plus élevée possibles du cycle d'oxydo-réduction entre les formes oxydée et réduite du système polymère-agent dopant.

Dans la demande de brevet FR-A-2596566, on a décrit une famille de polymères présentant, jusqu'à un certain degré, les propriétés susmentionnées. Ces polymères contiennent des unités récurrentes dérivées de 3-alkylthiophènes dont le substituant alkyle comprend de six à neuf atomes de carbone.

La présente invention vise à fournir une nouvelle famille de polymères conducteurs présentant les propriétés particulières susmentionnées à un degré encore bien plus élevé.

L'invention concerne à cet effet les polymères conducteurs d'électricité contenant des unités récurrentes dérivées de monomères hétérocycliques aromatiques à 5 chaînons substitués, en position 3 par rapport à l'hétéroatome, par un substituant de type éther lié à l'hétérocycle par un radical alkylène comprenant au moins deux atomes de carbone.

Les polymères conducteurs d'électricité selon l'invention dérivent généralement de monomères hétérocycliques aromatiques que l'on peut représenter par la formule générale ci-après : 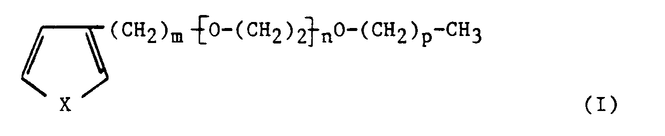
dans laquelle :
- X: représente un atome ou groupement choisi parmi les atomes de soufre et d'oxygène et le groupe -NH ;
- m: représente un nombre entier égal ou supérieur à 2 ;
- n: représente un nombre entier tel que 0 ≦ n ≦ 7 ;
- p: représente un nombre entier tel que 0 ≦ p ≦ 2 ;
la somme de n et de p étant au moins égale à 1.

Les polymères selon l'invention peuvent donc dériver du thiophène, du furane et du pyrrole substitués en position 3.

Le substituant est un groupement de type éther ; ce groupement, qui peut comprendre, comme le montre la formule (I), une à huit fonctions éthers, et de préférence deux ou trois fonctions éthers, séparées par des radicaux éthylène, est lié à l'hétérocycle par un radical alkylène comprenant au moins deux atomes de carbone, de préférence un radical éthylène ou triméthylène.

A titre d'exemples de polymères dérivés des monomères répondant à la formule générale (I) on peut citer les polymères dérivés des monomères dont le substituant comprend une fonction éther, tels que le 2′-méthoxy- et 2′-éthoxy-éthyl-3-thiophène, les 2′-méthoxy- et 2′-éthoxy-éthyl-3-furane et les 2′-méthoxy- et 2′-éthoxy-éthyl-3-pyrrole, par exemple les polymères dérivés de monomères dont le substituant comprend deux fonctions éther, tels que les (dioxa-3′,6′-heptyl) et (dioxa-3′,6′-octyl)-3-thiophène, les (dioxa-3′,6′-heptyl) et (dioxa-3′,6′-octyl)-3-furane et les (dioxa-3′,6′-heptyl) et (dioxa-3′,6′-octyl)-3-pyrrole, par exemple ; les polymères dérivés de monomères dont le substituant comprend plus de deux fonctions éther, tels que les (trioxa-3′, 6′,9'-décyl)-3-thiophène, -3-furane et -3-pyrrole et les (octaoxa-4′,7′,10′,13′,16′,19′,22′,25#MIN#-hexadodécyl)-3-thiophène, -3-furane et -3-pyrrole, par exemple.

Parmi tous les polymères mentionnés ci-dessus, on préfère les polymères dérivés du thiophène substitué en position 3 et dont le substituant comprend deux fonctions éther, c'est-à-dire les polymères dérivés du monomère de formule (I) dans laquelle X est un atome de soufre, m vaut 2 ou 3, n vaut 1 et p vaut 1 ou 0. Un composé tout particulièrement préféré, notamment parce qu'il conduit des polymères manifestant des propriétés de réversibilité du cycle d'oxydo-réduction exceptionnelles est le (dioxa-3′,6′-heptyl)-3-thiophène (DHT) (dont le polymère est dénommé ci-après "PDHT" par simplification).

Un autre objet de l'invention est constitué par des composés chimiques utilisables comme monomères permettant notamment d'obtenir de tels polymères dérivés du thiophène, substitué en position 3 et dont le substituant comprend deux fonctions éthers, choisis parmi les monomères de formule (I) pour lesquels m vaut 2 ou 3, n vaut 1 et p vaut 1 ou 0. De tels monomères peuvent être représentés par la formule générale (II) ci-après : 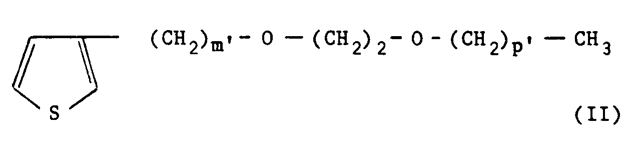
dans laquelle :
- m′: représente les nombres 2 ou 3
- p′: représente les nombres 0 ou 1.

Le monomère tout particulièrement préféré de cette catégorie est le composé pour lequel m' vaut 2 et p′ vaut 0 c-à-d le (dioxa-3′,6′-heptyl)-3-thiophène. Le DHT est préparé de préférence par réaction de (thiényl-3)-2′-éthanol avec le (chloro-2-éthyl) méthyléther dans le tétrahydrofurane en présence d'hydrure de sodium.

La température à laquelle est réalisée la réaction est généralement comprise entre 5 et 50°C, de préférence entre 10 et 30°C.

La pression à laquelle est réalisée la réaction est généralement comprise entre 1 et 4 bars, de préférence la réaction est réalisée à la pression atmosphérique.

La réaction est réalisée de préférence en présence d'un éther couronne et/ou d'hydrure de sodium.

La réaction est réalisée de préférence sous l'atmosphère d'un gaz inerte, tel que notamment l'argon ou l'azote, et en présence d'un solvant tel que notamment le tétrahydrofurane.

La réaction est réalisée dans tout réacteur ou appareillage permettant de réunir les conditions susmentionnées.

Les monomères utilisables pour fabriquer les polymères selon l'invention peuvent être synthétisés selon des méthodes connues, par exemple par réaction des (thiényl-3)-, (furanyl-3)- et (pyrrolo-3)-2′éthanol ou -3′propanol-1 avec l'halogénure d'oxyalkyle approprié.

La préparation des polymères selon l'invention peut s'effectuer par voie chimique, en présence d'oxydants tels que FeCl₃, par exemple ou par voie électrochimique. De préférence, on procède par polymérisation électrochimique, généralement dans une cellule d'électrolyse, par oxydation anodique du monomère au sein d'un solvant polaire et en présence d'électrolytes appropriés suivant des techniques conventionnelles (voir par exemple demande de brevet français FR-A-2527843 et F. Garnier et al., op. cit.).

Selon ces techniques, la concentration en monomères est généralement comprise entre 10⁻³ et 1 mole par litre de solvant. La température à laquelle est réalisé le procédé est généralement comprise entre 0 et 40°C et de préférence entre 4 et 30°C. La pression à laquelle est réalisé le procédé est généralement la pression atmosphérique.

Comme solvants, on utilise de préférence des solvants polaires possédant des propriétés dissolvantes à la fois vis-à-vis du monomère et de l'électrolyte choisi et stables dans le domaine des potentiels appliqués. Des exemples de solvants utilisables sont l'acétonitrile, le tétrahydrofurane, le chlorure de méthylène, le nitrobenzène et le carbonate de propylène.

Les électrolytes sont généralement choisis parmi les sels conducteurs de formule C⁺A⁻ dans laquelle C⁺ est un cation et dans laquelle A⁻ est un anion.

Le cation C⁺ est choisi de préférence parmi les ions alcalins, les ions R₄N⁺ et R₄P⁺ (R étant un radical alkyle, tel que les radicaux éthyle et butyle par exemple).

L'anion A⁻ est choisi de préférence parmi les ions ClO₄, AsF₆̅, SbF₆̅, SO₄²⁻, C₆H₅COO⁻, C₆H₅SO₃̅, BF₄̅, PF₆̅ et CF₃SO₃̅.

Des électrolytes typiques sont par exemple les fluorophosphates, tels que l'hexafluorophosphate de tétrabutylammonium, les fluoroborates, tels que le tétrafluoroborate de tétraéthylammonium et les perchlorates, tels que le perchlorate de lithium et le perchlorate de tétrabutylammonium.

La concentration en électrolyte est généralement comprise entre 10⁻³ et 1 mole par litre de solvant.

La cellule électrochimique au sein de laquelle peut s'effectuer la polymérisation des monomères selon l'invention peut fonctionner dans des conditions potentiostatiques ou galvanostatiques.

Dans le premier cas (contrôle potentiostatique), la cellule comprend, outre la source de courant externe, trois électrodes dont une électrode de référence de contrôle du potentiel.

Au cours de l'électrolyse, une couche de polymère se dépose sur l'élément conducteur utilisé comme anode de la cellule d'électrolyse. Cette anode peut être réalisée en un métal noble, tel que l'or ou le platine, ou en un autre métal, tel que le cuivre, doré ou platiné, le titane, le nickel ou en un verre conducteur (oxyde d'étain, oxydes d'indium - étain). Après l'électrolyse, on dispose donc en fait d'une électrode constituée par un corps conducteur enrobé par un film de polymère y adhérant et qui contient une certain proportion de l'anion provenant de l'électrolyte. Le polymère et l'anion forment ainsi un complexe à transfert de charges. La composition chimique du film de polymère peut être représentée par la formule empirique (M⁺A_{y}⁻)ₙ où M⁺ représente le monomère, A⁻ l'anion ou contre-ion, y la proportion en anion dans le polymère exprimée par unité monomérique (c'est-à-dire le taux de dopage) qui, dans le cas des polymères de l'invention peut atteindre la valeur de 0,5, et n le degré de polymérisation généralement impossible à déterminer aisément, compte tenu du caractère insoluble du polymère.

La polymérisation électrochimique du monomère s'effectuant sur l'anode de la cellule d'électrolyse, on ne peut obtenir directement une électrode recouverte d'un polymère dopé par des cations.

On peut, pour obtenir une telle cathode, se servir de l'anode obtenue précédemment et lui faire subir une double réduction. Une première réduction électrochimique est possible juste après la polymérisation en laissant l'anode dans la cellule d'électrolyse et en provoquant la décharge de la cellule. Cette décharge provoque l'extraction des anions "dopant" le polymère. On peut ensuite effectuer une seconde réduction sous atmosphère inerte, soit par voie chimique, soit par voie électrochimique. La voie chimique consiste à immerger le polymère dans une solution contenant les cations désirés. Ainsi, pour obtenir un polymère "dopé" par exemple par les cations Li⁺, Na⁺ ou K⁺ (dopage de type "n" auquel les polymères selon l'invention, surtout le PDHT, se prêtent particulièrement bien), on peut se servir par exemple d'une solution de naphtalène lithium, de naphtalène sodium ou de naphtalène potassium dans le tétrahydrofurane. La voie électrochimique consiste généralement à placer l'électrode en tant que cathode dans une cellule d'électrolyse contenant en solution les cations désirés. Les cations peuvent être par exemple des ions alcalins tels que ceux mentionnés ci-dessus, de préférence les cations Li⁺ ou K⁺, ou des ions complexes tels que (Bu)₄N⁺ ou (Et)₄N⁺ issus d'un électrolyte (de préférence LiClO₄, KPF₆, (Bu)₄NClO₄ et (Et)₄NClO₄) en solution dans un solvant tel que l'acétonitrile, le tétrahydrofurane ou le propylène carbonate. La concentration en électrolyte dans la solution est généralement comprise entre 10⁻³ et 1 mole pour 1 litre de solvant.

Les polymères conducteurs selon l'invention présentent un ensemble de propriétés tout à fait remarquables qui sont principalement :
- une réversibilité et une stabilité exceptionnelles du cycle d'oxydo-réduction entre leurs formes oxydées et réduites ; ainsi, notamment, pour le préféré d'entre ces polymères, le PDHT, la stabilité du cycle d'oxydo-réduction est telle qu'il peut subir jusqu'à 0,5.10⁷ cycles tout en conservant encore 90 % de la charge initiale ;
- une variation importante des caractéristiques spectrales obtenue avec une variation de potentiel très faible, ce qui rend intéressante et économique leur utilisation en tant que matériau électrochromique ;
- une bonne conductivité électrique, généralement comprise entre 10¹ et 10² S.cm⁻¹ ;
- des absorptions importantes dans le domaine des rayonnements infra-rouge proche et à haute fréquence.

Ces propriétés remarquables des polymères conducteurs selon l'invention les rendent particulièrement utilisables pour la réalisation de dispositifs électroconducteurs dont le principe de fonctionnement est basé sur ces propriétés, et qui constituent également un objet de la présente invention.

A titre d'exemples non limitatifs de dispositifs électroconducteurs contenant des polymères conducteurs dérivés des monomères hétérocycliques aromatiques substitués selon l'invention, on peut citer :
- les dispositifs électrochimiques de stockage d'énergie, tels que batteries d'accumulateurs et piles rechargeables ou non, dont les anodes (respectivement les cathodes) sont constituées d'électrodes revêtues de films desdits polymères dopés par des anions (respectivement des cations) ;
- les dispositifs électrochromiques basés sur la modification du spectre optique desdits polymères selon leur état électrochimique, qui se manifeste lors des cycles d'oxydation et de réduction des films de polymères déposés sur les anodes (respectivement les cathodes) de ces dispositifs lors de la charge et de la décharge ; à titre d'exemples de pareils dispositifs électrochimiques, on peut citer les écrans d'affichage (display), les dispositifs optoélectroniques, les mémoires et commutateurs optiques.

L'invention est illustrée par l'exemple non limitatif suivant.

### Exemple

### A. Préparation du monomère

A une solution de 100 mmole de (thiényl-3)-2′-éthanol (fourni par JANSSEN CHEMICA) dans 100 ml de tétrahydrofurane on ajoute 110 mmole de NaH à 60 % en dispersion dans l'huile et 200 mg d'un éther couronne commercialisé par ALDRICH sous la dénomination 18 C 6. La suspension est maintenue 2 heures à 20°C puis on ajoute en 15 minutes, 200 mmole de (chloro-2- éthyl)-méthyléther vendu par ALDRICH et portée à reflux 8 heures. La suspension ramenée à 20°C est traitée par HCl. Les phases organiques sont lavées à l'eau à neutralité puis évaporées sous vide. Le produit brut est purifié par chromatographie puis distillé sous vide.

### B. Synthèse du polymère

La synthèse du polymère est effectuée dans une cellule thermostatée à un compartiment contenant :
- 2.10⁻¹ mole de monomère préparé comme en A,
- 3.10⁻² mole d'hexafluorophosphate de tétrabutylammonium (fourni par Fluka)
- 25 ml de nitrobenzène distillé.

Les dépôts de polymère sont réalisés à 5°C, sous atmosphère d'argon, après dégazage de la solution par barbotage d'argon. Pour les caractérisations électrochimiques le polymère est déposé sur des électrodes de platine massif de surface de 0,07 et 0,7 cm² polies entre chaque expérience. Les quantités de charges utilisées varient de 20 à 100 mC/cm², ce qui correspond à des épaisseurs comprises entre 600 et 3000 Å. La cathode est constituée d'un fil de platine et l'électrode de référence est une électrode au calomel saturée.

Les films de polymère destinés aux études spectroscopiques et aux mesures de conductivité ont été synthétisés dans les mêmes conditions, l'électrode de travail étant constituée d'une lame de verre conducteur et la cathode d'une plaque d'aluminium. Les dépôts sont effectués en conditions galvanostatiques avec des densités de courant comprises entre 2 et 5 mA/cm². Les caractérisations spectroélectrochimiques ont été effectuées dans une cellule de spectrométrie de 1 cm de trajet optique équipée d'une cathode constituée d'un fil de platine et d'une électrode de référence constituée d'un fil d'argent.

Les films de polymère destinés aux tests de stabilité à long terme en réversibilité rédox ont été synthétisés sur une électrode de platine massif de 0,7 cm². Cette électrode est ensuite placée dans une cellule équipée d'une contre-électrode circulaire constituée d'un fil de platine et d'une électrode de référence constituée d'un fil d'argent. La cellule est équipée d'une fenêtre optique permettant la mesure du temps de réponse par réflexion à l'aide d'un laser hélium-néon (λ = 632,8 nm) et d'une photodiode GaAsP. Le cyclage rédox est effectué dans un milieu électrolytique constitué de carbonate de propylène contenant 5.10⁻¹ mole de perchlorate de lithium anhydre.

Les tests de dopage "n" ont été réalisés dans le carbonate de propylène contenant 5.10⁻² mole de perchlorate de tétraéthylammonium.

Les propriétés électrochimiques des polymères ont été mesurées à partir du voltammogramme cyclique enregistré au moyen d'un potentiostat PAR modèle 173 et à partir des pics d'intensité enregistrés. La vague anodique comprend deux pics d'oxydation : 0,4 et 0,8 V/SCE (électrode de référence au calomel). Pour ces deux systèmes le rapport I_{Pa}/I_{Pc} entre les intensités de courant d'oxydation (Iₚₐ) et de réduction (I_{Pc}) est d'environ 1.

Pour le premier système, la stabilité de la réversibilité rédox est, exprimée en pourcentage de charges encore échangées, après :

| | |
|---|---|
| 0,65.10⁶ cycles | 100 % |
| 1,5.10⁶ cycles | 96 % |
| 3,25.10⁶ cycles | 92 % |
| 0,5.10⁷ cycles | 90 % |

Le dopage "n" du polymère est évalué par une observation d'un système cathodique réversible entre -1,7 et -2,2 V/Ag

Les caractéristiques optiques et spectroscopiques sont l'importante absorption dans le rouge (bande d'absorption à λ = 600 nm) du polymère et l'absorption très faible au delà de 600 nm du polymère dédopé.

Par contre, le polymère dopé possède une bande d'absorption importante dans le rouge lointain et le proche infra-rouge.

Ce phénomène est à l'origine d'un comportement électrochrome particulier. En effet, lorsque l'on analyse la variation de l'intensité de la réflexion d'un faisceau laser (hélium-néon:λ = 632,8 nm) sur un film de polymère soumis à des impulsions de potentiel, on observe deux états stables pour des valeurs de potentiel appliqué allant jusqu'à 0.7 V/Ag tandis qu'au delà, on n'obtient plus qu'un seul état stable. Ce phènomène d'effet de seuil est utilisable pour des applications en opto-électronique.

## Revendications (Revendications pour l'(les) Etat(s) contractant(s) suivant(s): BE, CH, DE, FR, GB, IT, LI, NL, SE)

1. Polymères conducteurs d'électricité contenant des unités récurrentes dérivées de monomères hétérocycliques aromatiques à 5 chaînons substitués, uniquement en position 3 en rapport à l'hétéroatome, par un substituant de type éther lié à l'hétérocycle par un radical alkylène comprenant au moins deux atomes de carbone.

2. Polymères conducteurs selon la revendication 1, caractérisés en ce qu'ils dérivent de monomères hétérocycliques aromatiques représentés par la formule générale : 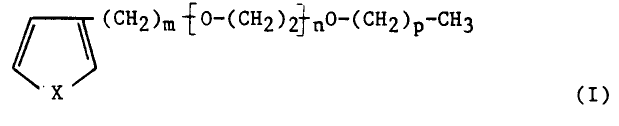 dans laquelle :
X représente un atome ou groupement choisi parmi les atomes de soufre et d'oxygène et le groupe -NH;
m représente un nombre entier égal ou supérieur à 2 ;
n représente un nombre entier tel que 0 ≦ n ≦ 7 ;
p représente un nombre entier tel que 0 ≦ p ≦ 2 ;
la somme de n et de p étant au moins égale à 1.

3. Polymères conducteurs selon la revendication 1 ou 2, caractérisés en ce que le monomère hétérocyclique aromatique dont ils dérivent est le thiophène substitué en position 3.

4. Polymères conducteurs selon l'une des revendications 1 à 3, caractérisés en ce que le monomère hétérocyclique aromatique dont ils dérivent est le (dioxa-3',6'-heptyl)-3-thiophène.

5. Procédé pour la fabrication de polymères électroconducteurs caractérisé en ce que l'on polymérise électrochimiquement, par oxydation anodique au sein d'un solvant polaire et en présence d'un électrolyte approprié, un monomère choisi parmi les hétérocycles aromatiques à cinq chaînons substitués, uniquement en position 3 par rapport à l'hétéroatome, par un substituant de type éther lié a l'hétérocycle par un radical alkylène comprenant au moins deux atomes de carbone.

6. Procédé selon la revendication 5, caractérisé en ce que le monomère est le (dioxa-3'-6'-heptyl)-3-thiophène.

7. Dispositifs électroconducteurs contenant un polymère selon l'une quelconque des revendications 1 à 4.

8. Dispositifs électrochimiques de stockage d'énergie dont les anodes (respectivement les cathodes) sont constituées d'électrodes revêtues de films de polymères selon l'une quelconque des revendications 1 à 4 dopés par des anions (respectivement des cations).

9. Dispositifs électrochromiques dont les anodes (respectivement les cathodes) sont revêtues de films de polymères selon l'une quelconque des revendications 1 à 4.

10. Dispositifs d'absorption des rayonnements infrarouge proche et à haute fréquence à base de polymères selon l'une quelconque des revendications 1 à 4.

11. Monomères utilisables pour obtenir des polymères conducteurs selon la revendication 3, caractérisés en ce qu'ils répondent à la formule générale : 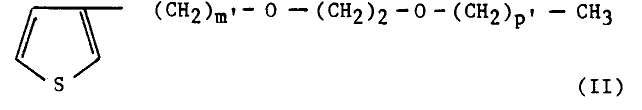 dans laquelle :
m' représente les nombres 2 ou 3 et
p' représente les nombres 0 ou 1.

12. Procédé pour la fabrication de monomères selon la revendication 11, caractérisé en ce qu'on fait réagir des (thiényl-3)-2'-éthanol ou -3'-propanol-1 avec un halogénure d'oxyalkyle approprié.

## Revendications (Revendications pour l'(les) Etat(s) contractant(s) suivant(s): AT, ES)

1. Procédé pour la fabrication de polymères électroconducteurs caractérisé en ce que l'on polymérise électrochimiquement, par oxydation anodique au sein d'un solvant polaire et en présence d'un électrolyte approprié, un monomère choisi parmi les hétérocycles aromatiques à cinq chaînons substitués, uniquement en position 3 par rapport à l'hétéroatome, par un substituant de type éther lie à l'hétérocycle par un radical alkylène comprenant au moins deux atomes de carbone à une température comprise entre 0 et 40°C et à la pression atmosphérique.

2. Procédé selon la revendication 1, caractérisé en ce que l'on utilise des monomères hétérocycliques aromatiques représentés par la formule générale 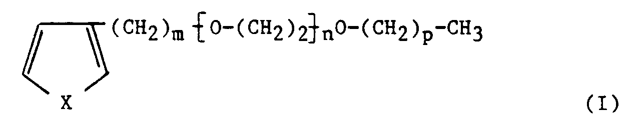 dans laquelle :
X représente un atome ou groupement choisi parmi les atomes de soufre et d'oxygène et le groupe -NH;
m représente un nombre entier égal ou supérieur à 2 ;
n représente un nombre entier tel que 0 ≦ n ≦ 7 ;
p représente un nombre entier tel que 0 ≦ p ≦ 2 ;
la somme de n et de p étant au moins égale à 1.

3. Procédé selon la revendication 1 ou 2, caractérisé en ce que le monomère hétérocyclique aromatique utilisé est le thiophène substitué en position 3.

4. Procédé selon la revendication 3, caractérisé en ce que l'on utilise des monomères répondant à la formule générale : 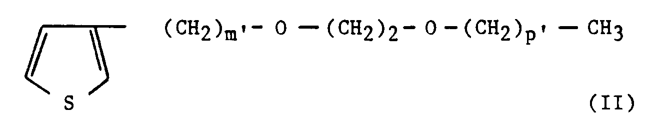 dans laquelle :
m′ représente les nombres 2 ou 3 et
p ′ représente les nombres 0 ou 1.

5. Procédé selon l'une quelconque des revendications 1 à 4, caractérisé en ce que le monomère hétérocyclique aromatique utilisé est le (dioxa-3',6'-heptyl)-3-thiophène.

6. Procédé pour la fabrication de monomères de formule générale : 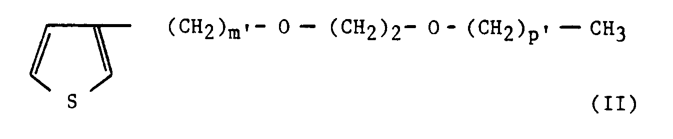 dans laquelle :
m' représente les nombres 2 ou 3 et
p' représente les nombres 0 ou 1,
caractérisé en ce qu'on fait réagir des (thiényl-3)-2'-éthanol ou -3'-propanol-1 avec un halogénure d'oxyalkyle approprié à une température comprise entre 5 et 50°C et à une pression comprise entre 1 et 4 bars.

7. Dispositifs électroconducteurs contenant un polymère selon l'une quelconque des revendications 1 à 5.

8. Dispositifs électrochimiques de stockage d'énergie dont les anodes (respectivement les cathodes) sont constituées d'électrodes revêtues de films de polymères selon l'une quelconque des revendications 1 à 5 dopés par des anions (respectivement des cations).

9. Dispositifs électrochromiques dont les anodes (respectivement les cathodes) sont revêtues de films de polymères selon l'une quelconque des revendications 1 à 5.

10. Dispositifs d'absorption des rayonnements infrarouge proche et à haute fréquence à base de polymères selon l'une quelconque des revendications 1 à 5.

## Claims (Claims for the following Contracting State(s): BE, CH, DE, FR, GB, IT, LI, NL, SE)

1. Electrically conductive polymers containing repeat units derived from 5-membered aromatic heterocyclic monomers substituted, solely in the 3 position relative to the heteroatom, with an ether-type substituent linked to the heterocyclic ring by an alkylene radical containing at least two carbon atoms.

2. Conductive polymers according to Claim 1, characterised in that they are derived from aromatic heterocyclic monomers denoted by the general formula: 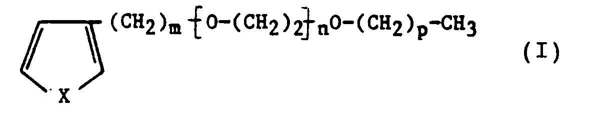 in which:
X denotes an atom or group chosen from sulphur and oxygen atoms and the -NH group,
m denotes an integer equal to or greater than 2,
n denotes an integer such that 0 ≦ n ≦ 7,
p denotes an integer such that 0 ≦ p ≦ 2,
the sum of n and of p being at least equal to 1.

3. Conductive polymers according to Claim 1 or 2, characterised in that the aromatic heterocyclic monomer from which they are derived is thiophene substituted in the 3 position.

4. Conductive polymers according to one of Claims 1 to 3, characterised in that the aromatic heterocyclic monomer from which they are derived is 3-(3',6'-dioxaheptyl)thiophene.

5. Process for the manufacture of electrically conductive polymers, characterised in that a monomer chosen from five-membered aromatic heterocyclic compounds substituted, solely in the 3 position relative to the heteroatom, by an ether-type substituent linked to the heterocyclic ring by an alkylene radical containing at least two carbon atoms is polymerised electrochemically by anodic oxidation in a polar solvent and in the presence of a suitable electrolyte.

6. Process according to Claim 5, characterised in that the monomer is 3-(3',6'-dioxaheptyl)thiophene.

7. Electrically conductive devices containing a polymer according to any one of Claims 1 to 4.

8. Electrochemical energy storage devices whose anodes (or cathodes) consist of electrodes coated with films of polymers according to any one of Claims 1 to 4, doped with anions (or cations).

9. Electrochromic devices whose anodes (or cathodes) are coated with polymer films according to any one of Claims 1 to 4.

10. Devices for the absorption of near infrared and high frequency radiations, based on polymers according to any one of Claims 1 to 4.

11. Monomers capable of being employed to obtain conductive polymers according to Claim 3, characterised in that they correspond to the general formula: 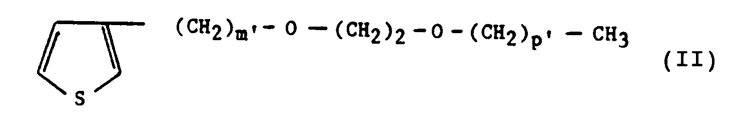 in which:
m' denotes the numbers 2 or 3, and
p' denotes the numbers 0 or 1.

12. Process for the manufacture of monomers according to Claim 11, characterised in that 2'-(3-thienyl)ethanol or the corresponding 3'-substituted 1-propanol is reacted with an appropriate oxyalkyl halide.

## Claims (Claims for the following Contracting State(s): AT, ES)

1. Process for the manufacture of electrically conductive polymers, characterised in that a monomer chosen from five-membered aromatic heterocyclic compounds substituted, solely in the 3 position relative to the heteroatom, by an ether-type substituent linked to the heterocyclic ring by an alkylene radical containing at least two carbon atoms, is polymerised electrochemically by anodic oxidation in a polar solvent and in the presence of a suitable electrolyte at a temperature of between 0 and 40°C and atmospheric pressure.

2. Process according to Claim 1, characterised in that aromatic heterocyclic monomers denoted by the general formula: 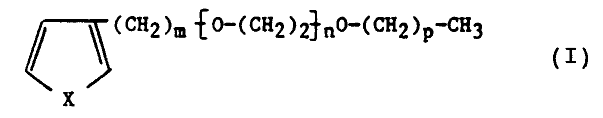 are used, in which:
X denotes an atom or group chosen from sulphur and oxygen atoms and the -NH group,
m denotes an integer equal to or greater than 2,
n denotes an integer such that 0 ≦ n ≦ 7,
p denotes an integer such that 0 ≦ p ≦ 2,
the sum of n and of p being at least equal to 1.

3. Process according to Claim 1 or 2, characterised in that the aromatic heterocyclic monomer used is thiophene substituted in the 3 position.

4. Process according to Claim 3, characterised in that the monomers used correspond to the general formula: 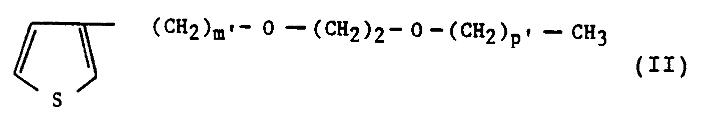 in which:
m' denotes the numbers 2 or 3 and
p' denotes the numbers 0 or 1.

5. Process according to any one of Claims 1 to 4, characterised in that the aromatic heterocyclic monomer employed is 3-(3',6'-dioxaheptyl)thiophene.

6. Process for the manufacture of monomers of general formula: 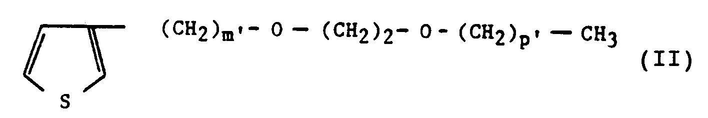 in which:
m' denotes the numbers 2 or 3 and
p' denotes the numbers 0 or 1,
characterised in that 2'-(3-thienyl)ethanol or the corresponding 3'-substituted 1-propanol is reacted with an appropriate oxyalkyl halide at a temperature of between 5 and 50°C and at a pressure of between 1 and 4 bars.

7. Electrically conductive devices containing a polymer according to any one of Claims 1 to 5.

8. Electrochemical energy storage devices whose anodes (or cathodes) consist of electrodes coated with films of polymers according to any one of Claims 1 to 5, doped with anions (or cations).

9. Electrochromic devices whose anodes (or cathodes) are coated with polymer films according to any one of Claims 1 to 5.

10. Devices for the absorption of near infrared and high frequency radiations, based on polymers according to any one of Claims 1 to 5.

## Patentansprüche (Patentansprüche für folgende(n) Vertragsstaat(en): BE, CH, DE, FR, GB, IT, LI, NL, SE)

1. Elektrizitätsleitfähige Polymere, die wiederkehrende Einheiten enthalten, abgeleitet von aromatischen, heterozyklischen Monomeren mit 5 Kettengliedern, die nur in der 3-Position bezüglich des Heteroatoms durch einen Substituenten vom Ether-Typ substituiert sind, der mit der heterozyklischen Verbindung durch einen Alkylenrest mit wenigstens 2 Kohlenstoffatomen verbunden ist.

2. Leitfähige Polymere nach Anspruch 1, dadurch gekennzeichnet, daß sie von aromatischen, heterozyklischen Monomeren, dargestellt durch die folgende allgemeine Formel, abgeleitet sind: 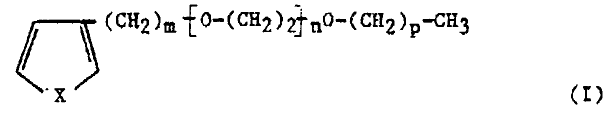 in der:
X ein Atom oder eine Gruppierung, ausgewählt unter den Schwefel- und Sauerstoffatomen und der Gruppe -NH darstellt;
m eine ganze Zahl gleich oder größer als 2 darstellt;
n eine ganze Zahl wie 0 ≦ n ≦ 7 darstellt;
p eine ganze Zahl wie 0 ≦ p ≦ 2 darstellt;
wobei die Summe von n und p wenigstens gleich 1 ist.

3. Leitfähige Polymere nach Anspruch 1 oder 2, dadurch gekennzeichnet, daß das aromatische heterozyklische Monomer, von dem sie abgeleitet sind, das in der 3-Position substituierte Thiophen ist.

4. Leitfähige Polymere nach einem der Ansprüche 1 bis 3, dadurch gekennzeichnet, daß das aromatische, heterozyklische Monomer, von dem sie abgeleitet sind, das (Dioxa-3',6'-heptyl)-3-thiophen ist.

5. Verfahren zur Herstellung von elektrizitätsleitfähigen Polymeren, dadurch gekennzeichnet, daß man elektrochemisch durch anodische Oxidation in einem polaren Lösungsmittel und in Anwesenheit eines geeigneten Elektrolyten, ein Monomer polymerisiert, das ausgewählt ist unter den aromatischen heterozyklischen Verbindungen mit 5 Kettengliedern , die nur in der 3-Position bezüglich des Heteroatoms durch einen Substituenten vom Ether-Typ substituiert sind, der mit der heterozyklischen Verbindung durch einen Alkylenrest mit wenigstens 2 Kohlenstoffatomen verbunden ist.

6. Verfahren nach Anspruch 5, dadurch gekennzeichnet, daß das Monomer das (Dioxa-3'-6'-heptyl)-3-thiophen ist.

7. Elektrizitätsleitfähige Vorrichtungen, die ein Polymer gemäß einem der Ansprüche 1 bis 4 enthalten.

8. Elektrochemische Vorrichtungen zum Speichern von Energie, bei denen die Anoden (bzw. die Kathoden) aus Elektroden gebildet sind, die mit Filmen aus Polymeren gemäß einem der Ansprüche 1 bis 4, dotiert durch Anionen (bzw. Kationen), überzogen sind.

9. Elektrochrome Vorrichtungen, deren Anoden (bzw. Kathoden) mit Filmen aus Polymeren gemäß einem der Ansprüche 1 bis 4 überzogen sind.

10. Vorrichtungen zur Absorption von nahen Infrarot- und Hochfrequenz-Strahlungen auf der Basis von Polymeren nach einem der Ansprüche 1 bis 4.

11. Monomere, die zum Erhalt von leitfähigen Polymeren nach Anspruch 3 verwendet werden können, dadurch gekennzeichnet, daß sie der allgemeinen Formel: 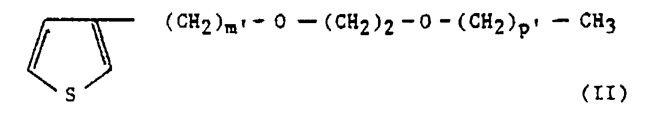 entsprechen, in der
m' die Zahlen 2 oder 3 darstellt und
p' die Zahlen 0 oder 1 darstellt.

12. Verfahren zur Herstellung von Monomeren nach Anspruch 11, dadurch gekennzeichnet, daß man (Thienyl-3)-2'-ethanol oder -3'-propanol-1 mit einem geeigneten Oxyalkylhalogenid reagieren läßt.

## Patentansprüche (Patentansprüche für folgende(n) Vertragsstaat(en): AT, ES)

1. Verfahren zur Herstellung von elektrizitätsleitfähigen Polymeren, dadurch gekennzeichnet, daß man elektrochemisch durch anodische Oxidation in einem polaren Lösungsmittel und in Anwesenheit eines geeigneten Elektrolyten, ein Monomer, das ausgewählt ist unter den aromatischen heterozyklischen Verbindungen mit 5 Kettengliedern, die nur in der 3-Position bezüglich des Heteroatoms durch einen Substituenten vom Ether-Typ substituiert sind, der mit der heterozyklischen Verbindung durch einen Alkylenrest mit wenigstens 2 Kohlenstoffatomen verbunden ist, bei einer Temperatur zwischen 0 und 40°C und bei atmosphärischem Druck polymerisiert.

2. Verfahren nach Anspruch 1, dadurch gekennzeichnet, daß man aromatische, heterozyklische Monomere verwendet, die durch die allgemeine Formel: 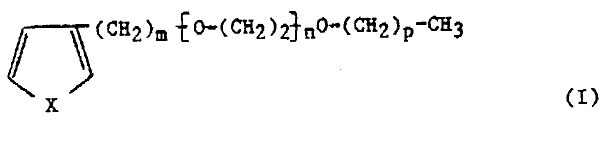 dargestellt sind, in der
X ein Atom oder eine Gruppierung, ausgewählt unter den Schwefel- und Sauerstoffatomen und der Gruppe -NH darstellt;
m eine ganze Zahl gleich oder größer als 2 darstellt;
n eine ganze Zahl wie 0 ≦ n ≦ 7 darstellt;
p eine ganze Zahl wie 0 ≦ p ≦ 2 darstellt;
wobei die Summe von n und p wenigstens gleich 1 ist.

3. Verfahren nach Anspruch 1 oder 2, dadurch gekennzeichnet, daß das verwendete aromatische, heterozyklische Monomer das in der 3-Position substituierte Thiophen ist.

4. Verfahren nach Anspruch 3, dadurch gekennzeichnet, daß man Monomere verwendet, die der allgemeinen Formel: 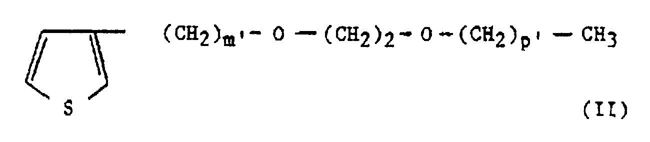 entsprechen, in der
m' die Zahlen 2 oder 3 darstellt und
p' die Zahlen 0 oder 1 darstellt.

5. Verfahren nach einem der Ansprüche 1 bis 4, dadurch gekennzeichnet, daß das verwendete aromatische, heterozyklische Monomer das (Dioxa-3',6'-heptyl)-3-thiophen ist.

6. Verfahren zur Herstellung von Monomeren der allgemeinen Formel: 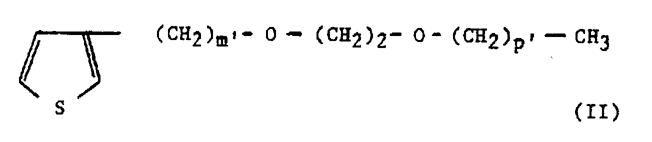 in der
m' die Zahlen 2 oder 3 darstellt und
p' die Zahlen 0 oder 1 darstellt,
dadurch gekennzeichnet, daß man (Thienyl-3)-2'ethanol oder -3'-propanol-1 mit einem geeigneten Oxyalkylhalogenid bei einer Temperatur zwischen 5 und 50°C bei einem Druck zwischen 1 und 4 bar reagieren läßt.

7. Elektrizitätsleitfähige Vorrichtungen, die ein Polymer gemäß einem der Ansprüche 1 bis 5 enthalten.

8. Elektrochemische Vorrichtungen zum Speichern von Energie, bei denen die Anoden (bzw. die Kathoden) aus Elektroden gebildet sind, die mit Filmen aus Polymeren gemäß einem der Ansprüche 1 bis 5, dotiert durch die Anionen (bzw. Kationen), überzogen sind.

9. Elektrochemische Vorrichtungen, deren Anoden (bzw. Kathoden) mit Filmen aus Polymeren gemäß einem der Ansprüche 1 bis 5 überzogen sind.

10. Vorrichtungen zur Absorption von nahen Infrarot- und Hochfrequenz-Strahlungen auf der Basis von Polymeren nach einem der Ansprüche 1 bis 5.
